(19) Europäisches Patentamt
European Patent Office
Office européen des brevets

(11) EP 1 267 853 B1

(12) EUROPEAN PATENT SPECIFICATION

(45) Date of publication and mention of the grant of the patent:
**08.09.2004 Bulletin 2004/37**

(21) Application number: **01931528.2**

(22) Date of filing: **13.03.2001**

(51) Int Cl.[7]: **A61K 31/00**, A61P 25/16

(86) International application number:
**PCT/EP2001/002896**

(87) International publication number:
**WO 2001/068065 (20.09.2001 Gazette 2001/38)**

# (54) COMPOSITIONS COMPRISING BLOCKERS OF L-DOPA RENAL CELL TRANSFER FOR THE TREATMENT OF PARKINSON'S DISEASE

ZUSAMMENSETZUNGEN, ENTHALTEND BLOCKER DES RENALEN ZELLTRANSFERS VON L-DOPA ZUR BEHANDLUNG DER PARKINSON-KRANKHEIT

COMPOSITIONS COMPRENANT DES AGENTS BLOQUANT LE TRANSFERT DE LA CELLULE RENALE L-DOPA DESTINES AU TRAITEMENT DE LA MALADIE DE PARKINSON

(84) Designated Contracting States:
**AT BE CH CY DE DK ES FI FR GB GR IE IT LI LU MC NL PT SE TR**
Designated Extension States:
**SI**

(30) Priority: **14.03.2000 GB 0006063**

(43) Date of publication of application:
**02.01.2003 Bulletin 2003/01**

(73) Proprietor: **Portela & Ca., S.A.**
**4745-457 S. Mamede do Coronado (PT)**

(72) Inventor: **Soares-Da-Silva, Patricio.**
**P-4150 Porto (PT)**

(74) Representative: **Harrison, Ivor Stanley et al**
**Withers & Rogers,**
**Goldings House, 2 Hays Lane**
**London SE1 2HW (GB)**

(56) References cited:
**WO-A-01/49281**
**WO-A-98/34646**
**GB-A- 2 348 371**
**US-A- 4 190 672**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 267 853 B1

## Description

The present invention relates to compositions for use in treating Parkinson's disease. In particular, it relates to the use of blockers of L-DOPA renal cell outward transfer as components of the said compositions.

Parkinson's disease (PD) is a chronic neurodegenerative disorder of unknown aetiology affecting to a great extent brain dopaminergic neurones originating in the *Substantia Nigra* and projecting to the *Striatum.* Clinically, PD patients show gradual motor impairment, which is more commonly manifested by tremor, rigidity and gait abnormalities. Subjects afflicted with PD show considerable motor improvement when administered L-DOPA, the precursor of the brain neurotransmitter dopamine, plus carbidopa or benserazide. The latter compounds are potent inhibitors of peripheral aromatic L-amino acid decarboxylase (AADC), and their administration is intended to abolish the conversion of L-DOPA to dopamine in peripheral tissues, therefore preventing the appearance of adverse effects related to the actions of dopamine in peripheral organs (cardiovascular and gastrointestinal). Inhibition of peripheral AADC is also accompanied by enhanced bioavailability of L-DOPA, which results in more effective replenishment of dopamine stores in unaffected brain dopaminergic neurones.

The relatively short half-life of L-DOPA is, however, a limitation in the maintenance therapy of PD patients, requiring the administration of multiple doses of L-DOPA. For such a reason, slow-release formulations of L-DOPA have recently been made available, in order to produce a more sustained motor improvement and reduce the number of daily administrations. This strategy is still of limited success because circulating L-DOPA is rapidly excreted into the urine, this route of elimination being of major importance. Although most L-DOPA appearing in the urine has its origin in filtered L-DOPA, a considerable amount of filtered L-DOPA is known to be reabsorbed along the nephron through sodium-dependent and sodium-independent amino acid transporters; at the level of proximal tubules, the absorbed L-DOPA can be easily converted to dopamine. In PD patients given L-DOPA plus an AADC inhibitor, the conversion of L-DOPA to dopamine in renal proximal tubules is blocked and most of the intracellular L-DOPA is believed to leave the cell through the apical cell border: this is a carrier-mediated transport system, the inhibition of which may lead to considerable accumulation of L-DOPA in the intracellular compartment.

This renal tubular L-DOPA outward transfer system was found to be sensitive to cyclosporine A (Pestana, et al., 1995, Br. J. Pharmacol. 115, 1349-1358), and further evidence suggested that P-glycoprotein was involved in the outward transfer of L-DOPA (Soares-da-Silva, et al., 1998, Br. J. Pharmacol. 123, 13-22; Soares-da-Silva & Serrão, 2000, J. Pharmacol. Exp. Ther. 293, 697-704). Because a major problem in the treatment of PD with L-DOPA is related to its short half-life and reduced bioavailability (Cederbaum, 1989, Clin. Neuropharmacol. 12, 147-166; Koller & Tolosa, 1998, Neurology, 50 (suppl. 6), S1-S48), it was thought that inhibitors of the renal tubular L-DOPA apical outward transfer (which promote its renal secretion) might reduce its elimination into the urine and enhance bioavailability. This would favour the movement of L-DOPA from the tubular epithelium to the renal interstitium and then back to circulation. Unfortunately, most P-glycoprotein inhibitors, which might prove beneficial in enhancing L-DOPA bioavailability in PD patients, are well-known cytotoxic agents or, if not toxic, the concentrations required. to produce inhibition of P-glycoprotein are unpracticable under *in vivo* conditions.

It is therefore an object of the present invention to provide compositions for the treatment of PD which comprise compounds capable of reducing the renal excretion of L-DOPA. It is also an object of the present invention to provide the use of at least one of the said compounds in the preparation of a medicament for the treatment of PD by sequential or simultaneous administration with L-DOPA.

Accordingly, the present invention provides compositions for the treatment of Parkinson's disease as described in the appended claims.

In another aspect, the invention provides the use of at least one blocker of L-DOPA renal cell outward transfer in the preparation of a medicament for the treatment of Parkinson's disease or movement disorders by sequential or simultaneous administration with L-DOPA, as described in the appended claims. The invention also provides the use of a composition comprising at least one such blocker compound in combination with L-DOPA in the preparation of a medicament for the treatment of Parkinson's disease, as described in the appended claims.

In a further aspect, the present invention provides at least one blocker of L-DOPA renal cell outward transfer, in combination with L-DOPA, for use in therapy as described in the appended claims.

In accordance with another aspect of the present invention, a method of treating Parkinson's disease is provided, the method comprising administering to a mammalian species in need of such treatment a therapeutically effective amount of a L-DOPA renal cell outward transfer blocking compound, in sequential or simultaneous combination with L-DOPA.

In addition, in accordance with a further aspect of the present invention, a method is provided for controlling movement of a Parkinsonian patient, wherein a therapeutically effective amount of a L-DOPA renal cell outward transfer blocking compound is administered to enhance the availability of sequentially or simultaneously administered L-DOPA to the brain and control movement.

The said blocking compound of the invention is selected from those described in any of appended claims 1 to 6.

The invention also provides, in another aspect , a method of both increasing the circulating levels of administered L-DOPA in a mammalian species and enhancing the availability of L-DOPA to the brain, the method comprising administering to the said species an effective amount of at least one of the blocking compounds as described above, in sequential or simultaneous combination with L-DOPA.

In addition to inhibiting renal tubular L-DOPA apical outward transfer the blocking compounds described herein simultaneously inhibit peripheral AADC. These compounds increase circulating levels of administered L-DOPA in plasma, inhibit AADC in the periphery, and enhance the availability of administered L-DOPA to the brain.

Preferably, the methods of the invention further comprise the administration of known inhibitors of AADC (such as benserazide or carbidopa) or catechol-o-methyl transferase (such as entacapone or tolcapone), either sequentially or simultaneously with the other active compounds. In addition, inert pharmaceutically acceptable carriers are admixed with the active compounds. The pharmaceutically acceptable carriers may be either solid or liquid. Solid form preparations include powders, tablets, dispersible granules and capsules. A solid carrier can be one or more substances which may also act as diluents, flavouring agents, solubilizers, lubricants, suspending agents, binders or tablet disintegrating agents; it may also be an encapsulating material.

Preferably, the pharmaceutical preparation is in unit dosage form, e.g. a packaged preparation, the package containing discrete quantities of preparation such as packeted tablets, capsules and powders in vials or ampoules.

The dosages may be varied depending on the requirements of the patient, the severity of the disease and the particular compound being employed. For convenience, the total daily dosage may be divided and administered in portions throughout the day. Determination of the proper dosage for a particular situation is within the skill of those in the medical art, but will preferably be in the range of about 40 µg to about 30,000 µg/kg per treatment in the case of the said blocking compounds.

Embodiments of the present invention will now be described in detail by way of example only and with reference to the appended drawings; of which:

Fig. 1 is a graph showing the effect of test compounds on the accumulation of L-DOPA in LLC-PK1 cells incubated for 6 min at 37°C with 2.5 µM of the substrate (L-DOPA).

Fig. 2 is a pair of graphs showing the effect of test compounds on the apical flux (Figure 2a) and cell accumulation (Figure 2b) of L-DOPA in LLC-PK1 cells incubated for 6 min at 37°C with 25 µM of the substrate (L-DOPA) applied from the basolateral cell border.

Fig. 3 is a graph showing the effect of test compounds on the decarboxylation of L-DOPA in LLC-PK1 cells incubated for 6 min at 37°C with 250 µM of the substrate (L-DOPA).

Fig. 4 is a graph showing the effect of increasing concentrations of benserazide upon brain, liver and kidney aromatic L-amino acid decarboxylase (AADC) activity measured in Vmax conditions (5 mM L-DOPA; 15 min incubation).

Fig. 5 is a graph showing the effect of resveratrol on levels of L-DOPA in plasma of rats given L-DOPA (12 mg/kg) plus benserazide (3 mg/kg) and resveratrol.

MATERIALS AND METHODS

IN VITRO STUDIES

Cell culture LLC-$PK_1$ cells, a porcine-derived proximal renal tubule epithelial cell line which retains several properties of proximal tubular epithelial cells in culture (Hull, R. N., et al., 1976, In Vitro 12, 670-677), were obtained from the American Type Culture Collection (Rockville, MD). LLC-$PK_1$ cells (ATCC CRL 1392; passages 198-206) and were maintained in a humidified atmosphere of 5% $CO_2$-95% air at 37°C and grown in Medium 199 (Sigma Chemical Company, St. Louis, Mo, USA) supplemented with 100 U/ml penicillin G, 0.25 µg/ml amphotericin B, 100 µg/ml streptomycin (Sigma), 3% foetal bovine serum (Sigma) and 25 mM *N*-2-hydroxyethylpiperazine-*N*'-2-ethanesulfonic acid (HEPES; Sigma). For subculturing, the cells were dissociated with 0.05% trypsin-EDTA, split 1:4 and subcultured in Costar flasks with 75- or 162-$cm^2$ growth areas (Costar, Badhoevedorp, The Netherlands). For uptake studies, the cells were seeded in collagen treated 24 well plastic culture clusters (internal diameter 16 mm, Costar) at a density of 40,000 cells per well or onto collagen treated 0.2 µm polycarbonate filter supports (internal diameter 12 mm Transwell, Costar) at a density 13,000 cells per well (2.0 x $10^4$ cells $cm^2$). The cell medium was changed every 2 days, and the cells reached confluence after 3-5 days of incubation. For 24 hours prior to each experiment, the cell medium was free of foetal bovine serum. Experiments were generally performed 2-3 days after cells reached confluency and 6-8 days

after the initial seeding and each $cm^2$ contained about 80 μg of cell protein.

Transport studies in LLC-$PK_1$ cells

On the day of the experiment, the growth medium was aspirated and the cells washed with Hanks' medium; thereafter, the cell monolayers were preincubated for 15 min in Hanks' medium at 37° C. The Hanks' medium had the following composition (mM): NaCl 137, KCl 5, $MgSO_4$ 0.8, $Na_2HPO_4$ 0.33, $KH_2PO_4$ 0.44, each 0.25, $MgCl_2$ 1.0, Tris HCl 0.15 and sodium butyrate 1.0, pH=7.4. The incubation medium also contained benserazide (1 μM) and tolcapone (1 μM) in order to inhibit the enzymes AADC and catechol-*O*-methyltransferase, respectively. Time course studies were performed in experiments in which cells were incubated with 0.5 μM substrate for 1, 3, 6 and 12 min. Saturation experiments were performed in cells incubated for 6 min with increasing concentrations of L-DOPA (2.5 to 250 μM). Test substances were applied from the apical side only, and were present during the preincubation and incubation periods. During preincubation and incubation, the cells were continuously shaken and maintained at 37°C. Apical uptake was initiated by the addition of 2 ml Hanks' medium with a given concentration of the substrate. Uptake was terminated by the rapid removal of uptake solution by means of a vacuum pump connected to a Pasteur pipette followed by a rapid wash with cold Hanks' medium and the addition of 250 μl of 0.2 mM perchloric acid. The acidified samples were stored at 4°C before injection into the high pressure liquid chromatograph for the assay of L-DOPA.

Previous studies have shown that some of the L-DOPA accumulated in LLC-$PK_1$ cells can leave the cell through apical outward transporter(s) (Soares-da-Silva, et al., 1998, Am. J. Physiol. 274, F243-F251), the inhibition of which leads to an increase in the cellular accumulation of L-DOPA (Soares-da-Silva, et al., 1998, Br. J. Pharmacol. 123, 13-22). Thus, in experiments designed to study the effect of drugs which increased the intracellular accumulation of L-DOPA, cells were incubated with 25 μM L-DOPA applied from the basal cell border and uptake (accumulation in the cell monolayer) and flux (transfer to opposite chamber) were measured over a 6 min period. Test drugs were applied from the apical side only, and were present during the preincubation and incubation periods. At the end of incubation, cells were placed on ice and the medium bathing the apical cell border was collected, acidified with perchloric acid and stored at 4°C till assayed for L-DOPA. The cells were washed with ice-cold Hanks' medium and added with 0.2 mM perchloric acid (100 μl and 500 μl in the upper and lower chambers, respectively); the acidified samples were stored at 4°C before injection into the high pressure liquid chromatograph for the assay of L-DOPA.

Decarboxylation studies

On the day of the experiment, the growth medium was aspirated and the cells (LLC-$PK_1$) washed with Hanks' medium; thereafter, the cell monolayers were preincubated for 15 min in Hanks' medium at 37°C. The Hanks' medium had the following composition (mM): NaCl 137, KCl 5, $MgSO_4$ 0.8, $Na_2HPO_4$ 0.33, $KH_2PO_4$ 0.44, $CaCl_2$ 0.25, $MgCl_2$ 1.0, Tris HCl 0.15 and sodium butyrate 1.0, pH=7.4. The incubation medium also contained pyridoxal phosphate (120 μM), tolcapone (1μM) and pargyline (100 μM). Saturation experiments were performed in cells incubated for 6 min with increasing concentrations of L-DOPA (2.5 to 250 μM). In experiments designed to study the effects of test compounds upon the decarboxylation of L-DOPA, cells were preincubated for 30 min in the presence of the compounds to be tested. After preincubation, cells were incubated for 6 min in Hanks' medium with 250 μM L-DOPA. The reaction was terminated by the addition of 250 μl of 0.2 mM perchloric acid. The acidified samples were stored at 4°C before injection into the high pressure liquid chromatograph for the assay of dopamine.

Cell viability

Cells cultured in plastic supports were preincubated for 15 min at 37°C and then incubated in the absence or the presence of L-DOPA and test compounds for a further 6 min. Subsequently the cells were incubated at 37°C for 2 min with trypan blue (0.2% w/v) in phosphate buffer. Incubation was stopped by rinsing the cells twice with Hanks' medium and the cells were examined using a Leica microscope. Under these conditions, more than 95% of the cells excluded the dye.

IN VIVO STUDIES

These experiments were designed to evaluate the effects of test compounds on the bioavailability and brain access of L-DOPA. Male Wistar rats weighing 170-280 g, kept two per cage under controlled environmental conditions (12 h light/dark cycle and room temperature 24 °C), were used in all experiments. At defined intervals, rats were killed by decapitation and their brains, livers and kidneys were removed and used to determine L-DOPA, dopamine and DOPAC.

Assay of AADC in rat tissues

In some experiments, AADC activity was determined in brain, liver and kidney under Vmax conditions (5 mM L-DOPA; 15 min incubation), as previously described (Soares-da-Silva, et al., 1994, Br. J. Pharmacol. 112,611-615). The reaction was stopped by the addition of 500 μl of 2 M perchloric acid and the preparations kept at 4°C for 60 min. The samples were then centrifuged (200 g, 2 min, 4°C) and 500 μl aliquots of the supernatant filtered on Spin-X filter tubes (Costar) were used for the assay of dopamine.

ASSAY OF L-DOPA, DOPAMINE AND AMINE METABOLITES

L-DOPA, dopamine and amine metabolites (DOPAC and HVA) were quantified by means of high pressure liquid chromatography with electrochemical detection, as previously reported (Soares-da-Silva and Garrett, 1990, Neuropharmacol. 29, 869-874; Soares-da-Silva, et al., 1998, Am. J. Physiol. 274, F243-F251). The high pressure liquid chromatograph system consisted of a pump (Gilson model 302; Gilson Medical Electronics, Villiers le Bel, France) connected to a manometric module (Gilson model 802 C) and a stainless-steel 5 μm ODS column (Biophase; Bioanalytical Systems, West Lafayette, IN) of 25 cm length; samples were injected by means of an automatic sample injector (Gilson model 231) connected to a Gilson dilutor (model 401). The mobile phase was a degassed solution of citric acid (0.1 mM), sodium octylsulphate (0.5 mM), sodium acetate (0.1 M), EDTA (0.17 mM), dibutylamine (1 mM) and methanol (8% v/v), adjusted to pH 3.5 with perchloric acid (2 M) and pumped at a rate of 1.0 ml min-1. The detection was carried out electrochemically with a glassy carbon electrode, an Ag/AgCl reference electrode and an amperometric detector (Gilson model 141); the detector cell was operated at 0.75 V. The current produced was monitored using the Gilson 712 HPLC software. The lower limits for detection of L-DOPA, dopamine, DOPAC, 3-MT and HVA ranged from 350 to 500 fmol.

DATA ANALYSIS

$K_m$ and $V_{max}$ values for the uptake of L-DOPA, as determined in saturation experiments, were calculated from non-linear regression analysis using the GraphP.ad Prism statistics software package (Motulsky, P. Spannard, R. Neubig. *GraphPad Prism (version 1*.0). San Diego, USA: GraphPad Prism Software Inc., 1994).

Apical fractional outflow was calculated using the expression

$$\text{L-DOPA}^{\text{apical fluid}} / (\text{L-DOPA}^{\text{apical fluid}} + \text{L-DOPA}^{\text{cell}})$$

where $\text{L-DOPA}^{\text{apical fluid}}$ indicates the amount of L-DOPA (in nmol/mg protein) which reached the apical chamber and $\text{L-DOPA}^{\text{cell}}$ (in nmol/mg protein) indicates the amount of L-DOPA accumulated in the cell monolayer.

Arithmetic means are given with S.E.M.. Statistical analysis was performed by one-way analysis of variance (ANOVA) followed by Newman-Keuls test for multiple comparisons. A P value less than 0.05 was assumed to denote a significant difference.

RESULTS

The accumulation of a non-saturating concentration (0.5 μM) of L-DOPA, in time course experiments, increased linearly with time for several minutes. At 6 min incubation, when uptake was linear with respect to time, and considering intracellular water as 7.0±0.7 μl/mg protein (Soares-da-Silva, et al., 1998, Am. J. Physiol. 274, F243-F251), the intracellular . L-DOPA concentration was 4.0±0.4 μM at a medium concentration of 0.5 μM. This represented a cell concentration of L-DOPA that was 8.0±0.8 times higher than the corresponding medium concentration. In experiments designed to determine the kinetic parameters of the L-DOPA apical transporter, the cells were incubated for 6 min with increasing concentrations (1 to 250 μM) of the substrate. Non-linear analysis of the saturation curve for L-DOPA revealed a $K_m$ value (in μM) of 47±8 and a $V_{max}$ value (in pmol mg protein/6 min) of 3069±224.

In order to evaluate the metabolic requirements for L-DOPA uptake, cells were incubated at 4°C. The effect of reducing temperature from 37° to 4°C during preincubation and incubation was a marked decrease (77±2% reduction) in L-DOPA (2.5 μM) accumulation.

Reducing extracellular sodium (from 140 mM to 70, 35 and 0 mM) did not affect the accumulation of L-DOPA. Moreover, in the absence of extracellular sodium (replaced by an equimolar concentration of choline), $K_m$ and $V_{max}$ values for L-DOPA were similar to those observed in the presence of sodium. N-(methylamino)-isobutyric acid (MeAIB) failed to affect the uptake of L-DOPA, whereas 2-aminobicyclo(2,2,1)-heptane-2-carboxylic acid (BHC) produced a concentration-dependent inhibition of L-DOPA uptake ($IC_{50}$=407 μM). The inhibitory effect of 1 mM BHC on the accumulation of L-DOPA was of the competitive type, as evidenced by the increase in $K_m$ (130±19 μM) but not $V_{max}$ (3783±244 pmol/mg protein/6 min) values for L-DOPA uptake. Taken together, these results suggest that the apical inward transfer of L-DOPA may be promoted through the BHC-sensitive and sodium-independent L-type amino acid transporter (Audus and Borchardt, 1986, J. Neurochem. 47,484-488).

Blockers of L-DOPA renal cell outward transfer were found to increase the accumulation of L-DOPA (2.5 μM) in a concentration dependent manner with $EC_{50}$'s from 6 to 339 μM (Figure 1). Pretreatment of cells with test compounds was found to significantly increase (P<0.05) the maximal accumulation ($V_{max}$) of increasing concentrations of L-DOPA without significant changes in $K_m$ values.

To demonstrate that increased accumulation of L-DOPA induced by test compounds was due to a reduced outward transfer of intracellular (recently accumulated) L-DOPA, cells cultured in polycarbonate filters were incubated with L-DOPA (25 μM) applied from the basal cell border and the basal-to-apical flux of L-DOPA was measured. As shown in Figure 2, test compounds markedly reduced the basal-to-apical flux of L-DOPA and increased the accumulation of L-DOPA in the cell, clearly indicating their effect as blockers of L-DOPA renal cell outward transfer.

The next series of experiments was intended to evaluate the inhibitory potency of test compounds upon AADC. For this purpose, LLC-$PK_1$ cells were preincubated for 30 min with increasing concentrations of test compounds or benserazide; thereafter cells were incubated with a concentration of L-DOPA approaching saturation (250 μM). As shown in Figure 3, test compounds were found to inhibit AADC activity in a concentration-dependent manner, as measured by the conversion of L-DOPA to dopamine. $IC_{50}$'s varied from 0.07μM (for benserazide) up to 393 μM (for the less potent).

In experiments conducted in vivo, rats were given the test compounds by the oral route 30 min before the administration of L-DOPA or L-DOPA plus benserazide. Since high doses of benserazide may result in inhibition of AADC in brain (Da Prada, et al., 1987, Eur. Neurol: 27, 9-20), preliminary experiments were carried out in order to determine the dose of benserazide which did not affect AADC activity in brain.

As shown in Figure 4, the dose of benserazide that produced maximal inhibition of liver and kidney AADC, being devoid of effects upon brain AADC, was 3 mg/kg benserazide, which is in full agreement with data from others (Da Prada, et al., 1987, Eur. Neurol. 27, 9-20). In order to conform with the proportion of L-DOPA/benserazide usually used in humans (4:1 ratio), the dose of L-DOPA used was set at 12 mg/kg.

When resveratrol was given orally to non- anaesthetised rats 30 min before L-DOPA (12 mg/kg) plus benserazide (3 mg/kg) administration, the result was a marked increase in tissue levels of L-DOPA, dopamine and amine metabolites in brain (Table 1). Increases in plasma levels of L-DOPA (Figure 5) and increases in tissue levels of L-DOPA and dopamine in the kidney (Table 1) accompanied these effects. It is possible that the AADC inhibitory effect of resveratrol did not contribute to a great extent to enhance the availability of L-DOPA. Firstly, dopamine levels in kidney in rats given test resveratrol were greater than in corresponding controls (rats given L-DOPA plus benserazide). Secondly, the inhibitory effect of 30 mg/kg of resveratrol upon liver and kidney AADC activity (liver, 48% reduction; kidney 38% reduction) was markedly less than that observed for benserazide (Figure 4).

Taken together the data mentioned above demonstrate that blockers of L-DOPA renal cell outward transfer result in higher levels of L-DOPA in plasma and enhance its availability to the brain.

Table 1.

Levels (in ng/g) of L-3,4-dihydroxyphenylalanine (L-DOPA), dopamine (DA), 3,4-dihydroxyphenylacetic acid (DOPAC) and homovanillic acid (HVA) in brain and kidney after administration of vehicle (0.5% carboxymethylcellulose, 4 ml/kg), L-DOPA (L, 12 mg/kg), L-DOPA (L, 12 mg/kg) plus benserazide (B, 3 mg/kg) and resveratrol (R) plus L-DOPA (L; 12 mg/kg) plus benserazide (B, 3 mg/kg). Resveratrol was administered 30 min before L-DOPA + benserazide, and rats were killed 60 min after L-DOPA + benserazide administration.

| **Brain** | **L-DOPA** | **DA** | **DOPAC** | **HVA** |
|---|---|---|---|---|
| Vehicle | 5.6±1.6 | 292.0±120.0 | 33.3±12.5 | 308.St61.4 |
| L | 9.6±1.0 | 201.0±19.6 | 24.3±2.4 | 320.6±37.4 |
| L+B | 116.3±23.5 | 222.0±16.8 | 132.0±13.7 | 2435.2±308.5 |
| R (0.3mg/kg)+L+B | 146.8±14.9 | 280.6±36.6 | 192.7±13.8 | 3975.7±62.1 * |
| R (1.0 mg/kg) + L + B | 143.5±29.1 | 221.9±41.5 | 149.9±15.8 | 3068.6±234.4 |
| R (3.0 mg/kg) + L + B | 180.9+26.3 * | 412.5±79.7 * | 221.2±33.8 * | 3026.5±337.2 |

Significantly different from corresponding values in rats treated with L-DOPA (L, 12 mg/kg) plus benserazide (B, 3 mg/kg) (* P<0.05).

Table 1. (continued)

| Brain | L-DOPA | DA | DOPAC | HVA |
|---|---|---|---|---|
| R (10.0 mg/kg) + L + B | 208.4±21.8 * | 531.9±41.6 * | 316.6±20.9 * | 2449.5±93.5 |
| R (30.0 mg/kg) + L + B | 294.0±52.1* | 403.2±99.2* | 257.8±51.1 * | 3324.8±529.4 |
| | | | | |
| **Kidney** | **L-DOPA** | **DA** | **DOPAC** | **HVA** |
| Vehicle | 14.2+1.1 | 6.2+0.7 | 0.3+0.3 | 264.7±41.0 |
| L | 17.4+2.1 | 120.8+27.5 | 84.3+23.3 | 10235.5±3772.9 |
| L + B | 437.7+50.4 | 470.9+44.0 | 332.9+53.8 | 17591.0±2286.6 |
| R(0.3mg/kg)+L+B | 857.4+103.3 * | 685.4+148.4 | 564.2+183.0 | 27169.4±2617.0 * |
| R (1.0 mg/kg) + L + B | 739.3+144.7 * | 622.5+61.5 * | 598.3+144.5 | 26254.3±3677.0 |
| R(3.0mg/kg)+L+B | 1016.2+121.5 * | 615.0+60.8 * | 420.4+44.4 * | 29094.8±3342.3 * |
| R (10.0 mg/kg) + L + B | 949.3+126.4 * | 710.3+67.2 * | 476.3+52.8 * | 25779.8±3941.7 |
| R (30.0 mg/kg) + L + B | 1198.6+225.1 * | 864.4+316.1 * | 731.9+427.3 | 27923.6±10686.2 |

Significantly different from corresponding values in rats treated with L-DOPA (L, 12 mg/kg) plus benserazide (B, 3 mg/kg) (* P<0.05).

## Claims

**1.** A composition for the treatment of Parkinson's disease, the composition comprising, in combination with L-DOPA, at least one compound selected from derivatives of phenyl benzopyran, trans-stilbene derivatives or 3-(4-hydroxyphenyl)-1-(2,4,6-trihydroxyphenyl)-1-propanone (phloretin) for blocking L-DOPA renal cell outward transfer, which enhances the availability of L-DOPA to the brain.

**2.** A composition according to claim 1, in which the derivative of phenyl benzopyran comprises a flavonoid compound.

**3.** A composition according to claim 2, in which the flavonoid compound has the general formula:

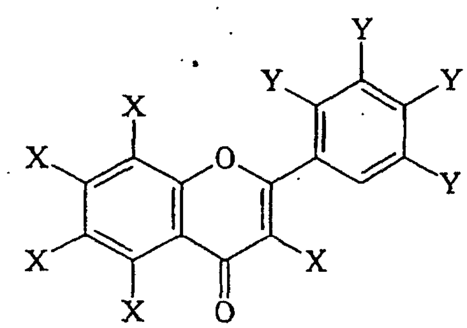

in which the X groups are the same or different and are selected from H and OH, the Y groups are the same or different and are selected from H and OR where R represents H, $CH_3$ and $CH_2$-Ph, including the corresponding 3-$C_6H_5(Y_4)$ derivatives.

**4.** A composition according to claim 1, in which the trans-stilbene derivative has the general formula:

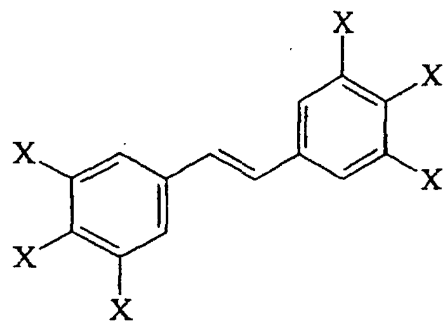

in which the X groups are the same or different and are H or OH.

**5.** A composition according to claim 1, in which the blocker compound is selected from:

5,7-dihydroxy-2-(4-methoxyphenyl)-4H-1-benzopyran-4-one (acacetin), 5,7-dihydroxy-2-(4-hydroxyphenyl)-4H-1-benzopyran-4-one (apigenin), 5,6,7-trihydroxy-2-phenyl-4H-1-benzopyran-4-one (baicalein), 5,7-dihydroxy-2-phenyl-4H-1-benzopyran-4-one (chrysin), ([2R,3R])-2-[3,4-dihydroxyphenyl]-3,4-dihydro-1[2H]benzopyran-3,5,7-triol ((-)-epicatechin), 2-(3,4-dihydroxyphenyl)-3,7-dihydroxy-4H-1-benzopyran-4-one (fisetin), 5,7-dihydroxy-3-(4-hydroxyphenyl)-4H-1-benzopyran-4-one (genistein), 3,5,7-trihydroxy-2-(4-hydroxyphenyl)-4H-1-benzopyran-4-one (kaempferol), 2-(2,4-dihydroxyphenyl)-3,5,7-trihydroxy-4H-1-benzopyran-4-one (morin), 3,5,7-trihydroxy-2-(3,4,5-trihydroxyphenyl)-4H-1-benzopyran-4-one (myricetin), 3-(4-hydroxyphenyl)-1-(2,4,6- trihydroxyphenyl)-1-propanoae (phloretin), 2-(3,4-dihydroxyphenyl)-3,5,7-trihydroxy-4H-1-benzopyran-4-one (quercetine), 2-(3,4-dihydroxyphenyl)-3,5,6,7-tetrahydroxy-4H-1-benzopyran-4-one (quercetagetin), 4'-benzyloxy-3',3'-dimethoxy-3,5,7-trihydioxyflavone, 3,7-dihydroxy-3',4',5'-trimethoxyflavone, 3',4',7,8-tetrahydroxyflavone, 3,5,7-trihydroxy-3',4',5'-trimethoxyflavone, 3,3',4,5'-tetrahydroxy-trans-stilbene (piceatannol), trans-4-styryl and 3,4'5-trihydroxy-trans-stilbene (resveratrol).

**6.** A composition according to any of claims 1, 4 or 5, in which the blocker compound is resveratrol.

**7.** A composition according to any preceding claim further comprising an inhibitor of the enzyme amino acid decarboxylase and/or an inhibitor of the enzyme catechol-o-methyl transferase.

**8.** A composition according to claim 7 in which the said amino acid decarboxylase inhibitor comprises benserazide or carbidopa and the said catechol-o-methyl transferase inhibitor comprises entacapone or tolcapone.

**9.** A composition according to any preceding claim, the composition further comprising inert, pharmaceutically acceptable excipients.

**10.** The use of a composition according to any preceding claim in the preparation of a medicament for the treatment of, or prevention of worsening of, any form of Parkinson's disease.

**11.** At least one blocker of L-DOPA renal cell outward transfer selected from derivatives of phenyl benzopyran, trans-stilbene derivatives or 3-(4-hydroxyphenyl)-1-(2,4,6-trihydroxyphenyl)-1-propanone (phloretin); in combination with L-DOPA, for use in therapy either by sequential or simultaneous administration.

**12.** At least one blocker compound according to claim 11, where the at least one blocker compound is selected from those described in any of claims 2 to 6.

**13.** The use of at least one blocker of L-DOPA renal cell outward transfer selected from derivatives of phenyl benzopyran, trans-stilbene derivatives or 3-(4-hydroxyphenyl)-1-(2,4,6-trihydroxyphenyl)-1-propanone (phloretin) in the

preparation of a medicament for the treatment of, or prevention of worsening of, any form of Parkinson's disease by sequential or simultaneous administration with L-DOPA.

**14.** The use of at least one blocker of L-DOPA renal cell outward transfer selected from derivatives of phenyl benzopyran, trans-stilbene derivatives or 3-(4-hydroxyphenyl)-1-(2,4,6-trihydroxyphenyl)-1-propanone (phloretin) in the preparation of a medicament for the treatment of movement disorders by modification of the net dopaminergic activity of the nigrostriatal pathway and by sequential or simultaneous administration with L-DOPA.

**15.** The use of at least one L-DOPA renal cell outward transfer blocking compound selected from derivatives of phenyl benzopyran, trans-stilbene derivatives or 3-(4-hydroxyphenyl)-1-(2,4,6-trihydroxyphenyl)-1-propanone (phloretin) in the preparation of a medicament for increasing the circulating levels of administered L-DOPA in a subject.

**16.** The use of at least one L-DOPA renal cell outward transfer blocking compound selected from derivatives of phenyl benzopyran, trans-stilbene derivatives or 3-(4-hydroxyphenyl)-1-(2,4,6-trihydroxyphenyl)-1-propanone (phloretin) in the preparation of a medicament for enhancing the availability of administered L-DOPA to the brain of a subject.

**17.** Use according to any of claims 13 to 16 where the at least one blocker compound is selected from those described in any of claims 2 to 6.

**18.** Use according to any of claims 13 to 17 wherein the blocker compound is present in an amount which will provide about 40 to about 30,000 μg/kg per dose.

**19.** Use according to any of claims 13 to 18 wherein the treatment also comprises the simultaneous or sequential administration of an inhibitor of amino acid decarboxylase or an inhibitor of catechol-o-methyl transferase.

**20.** Use according to claim 19 wherein the decarboxylase inhibitor comprises benserazide or carbidopa and the methyltransferase inhibitor comprises entacapone or tolcapone.

**21.** The use of at least one blocker compound selected from those described in any of claims 1 to 6 in the preparation of a medicament for the treatment of Parkinson's disease by blocking the peripheral decarboxylation of sequentially or simultaneously administered L-DOPA.

## Patentansprüche

**1.** Eine Zusammensetzung für die Behandlung der Parkinson Krankheit, wobei die Zusammensetzung, in Verbindung mit L-DOPA, wenigstens eine Verbindung umfasst, ausgewählt aus Derivaten von Phenylbenzopyran, trans-Stilben-Derivaten oder 3-(4-Hydroxyphenyl)-1-(2,4,6-trihydroxyphenyl)-1-propanon (Phloretin) zum Hemmen des Transports von L-DOPA aus den Nierenzellen, was die Verfügbarkeit von L-DOPA für das Gehirn verbessert.

**2.** Eine Zusammensetzung gemäß Anspruch 1, bei der das Phenylbenzopyranderivat eine Flavonoidverbindung umfasst.

**3.** Eine Zusammensetzung gemäß Anspruch 2, bei der die Flavonoidverbindung die allgemeine Formel aufweist:

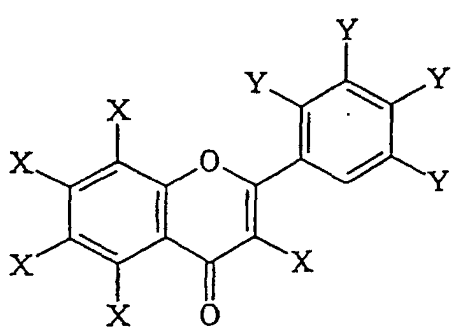

bei der die X Gruppen gleich oder unterschiedlich sind und ausgewählt werden aus H und OH, die Y Gruppen gleich oder unterschiedlich sind und ausgewählt werden aus H und OR, wobei R H, $CH_3$ und $CH_2$-Ph darstellt, einschließlich der entsprechenden 3-$C_6H_5$ ($Y_4$) Derivate.

**4.** Eine Zusammensetzung gemäß Anspruch 1, bei der das trans-Stilben-Derivat die allgemeine Formel aufweist:

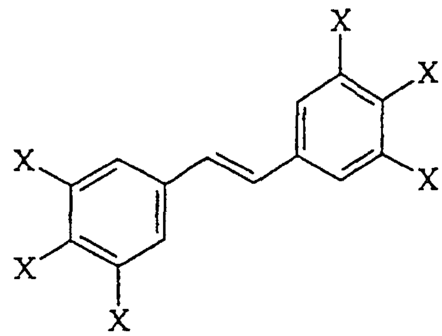

bei der die X Gruppen gleich oder unterschiedlich sind und H oder OH sind.

**5.** Eine Zusammensetzung gemäß Anspruch 1, bei der die hemmende Verbindung ausgewählt wird aus: 5,7-Dihydroxy-2-(4-methoxyphenyl)-4H-1-benzopyran-4-on (Acazetin),
5,7-Dihydroxy-2-(4-hydroxyphenyl)-4H-1-benzopyran-4-on (Apigenin),
5,6,7-Trihydroxy-2-phenyl-4H-1-benzopyran-4-on (Baicalein),
5,7-Dihydroxy-2-phenyl-4H-1-benzopyran-4-on (Chrysin),
([2R,3R])-2-[3,4-Dihydroxyphenyl]-3,4-dihydro-1[2H]benzopyran-3,5,7-triol((-)-Epicatechin),
2-(3,4-Dihydroxyphenyl)-3,7-dihydroxy-4H-1-benzopyran-4-on (Fisetin),
5,7-Dihydroxy-3-(4-hydroxyphenyl)-4H-1-benzopyran-4-on (Genistein),
3,5,7-Trihydroxy-2-(4-hydroxyphenyl)-4H-1-benzopyran-4-on (Kämpferol),
2-(2,4-Dihydroxyphenyl)-3,5,7-trihydroxy-4H-1-benzopyran-4-on (Morin),
3,5,7-Trihydroxy-2-(3,4,5-trihydroxyphenyl)-4H-1-benzopyran-4-on (Myricetin),
3-(4-Hydroxyphenyl)-1-(2,4,6-trihydroxyphenyl)-1-propanon (Phloretin),
2-(3,4-Dihydroxyphenyl)-3,5,7-trihydroxy-4H-1-benzopyran-4-on (Quercetin),
2-(3,4-Dihydroxyphenyl)-3,5,6,7-tetrahydroxy-4H-1-benzopyran-4-on (Quercetagetin),
4'-Benzyloxy-3',5'-dimethoxy-3,5,7-trihydroxyflavon,
3,7-Dihydroxy-3',4',5'-trimethoxyflavon,
3',4',7,8-tetrahydroxyflavon,
3,5,7-Trihydroxy-3',4',5'-trimethoxyflavon,
3,3',4,5'-Tetrahydroxy-trans-Stilben (Piceatannol), trans-4-Styrylphenol, und
3,4',5-Trihydroxy-trans-Stilben (Resveratrol).

**6.** Eine Zusammensetzung gemäß einem der Ansprüche 1, 4 oder 5, bei der die hemmende Verbindung Resveratrol ist.

**7.** Eine Zusammensetzung gemäß einem der vorangehenden Ansprüche, die des Weiteren einen Inhibitor des Enzyms Aminosäuredecarboxylase und/oder einen Inhibitor des Enzyms Catechol-O-Methyltransferase umfasst.

**8.** Eine Zusammensetzung gemäß Anspruch 7, bei der der Aminosäuredecarboxylaseinhibitor Benserazid oder Carbidopa umfasst und der Catechol-O-Methyltransferaseinhibitor Entacapon oder Tolcapon umfasst.

**9.** Eine Zusammensetzung gemäß einem der vorangehenden Ansprüche, wobei die Zusammensetzung ferner inerte, pharmazeutisch akzeptable Arzneimittelträger umfasst.

**10.** Die Verwendung einer Zusammensetzung gemäß einem der vorangehenden Ansprüche bei der Herstellung eines Medikamentes zur Behandlung der Parkinson Krankheit, oder zur Verhinderung der Verschlimmerung von jeglicher Form der Parkinson Krankheit.

**11.** Wenigstens ein Hemmer des Transports von L-DOPA aus den Nierenzellen, ausgewählt aus Phenylbenzopyranderivaten, trans-Stilben-Derivaten, oder 3-(4-Hydroxyphenyl)-1-(2,4,6-trihydroxyphenyl)-1-propanon (Phloretin), in Verbindung mit L-DOPA, zur therapeutischen Verwendung, entweder durch Verabreichung in einer Abfolge oder durch gleichzeitige Verabreichung.

**12.** Wenigstens eine hemmende Verbindung gemäß Anspruch 11, wobei die wenigstens eine hemmende Verbindung aus denjenigen, die in einem der Ansprüche 2 bis 6 beschrieben sind, ausgewählt ist.

**13.** Die Verwendung von wenigstens einem Hemmer des Transports von L-DOPA aus den Nierenzellen, ausgewählt aus Phenylbenzopyranderivaten, trans-Stilben-Derivaten oder 3-(4-Hydroxyphenyl)-1-(2,4,6-trihydroxyphenyl)-1-propanon (Phloretin) bei der Herstellung eines Medikaments zur Behandlung oder zur Verhinderung der Verschlimmerung von jeglicher Form der Parkinson Krankheit durch Verabreichung in einer Abfolge oder durch gleichzeitige Verabreichung mit L-DOPA.

**14.** Die Verwendung von wenigstens einem Hemmer des Transports von L-DOPA aus den Nierenzellen, ausgewählt aus Phenylbenzopyranderivaten, trans-Stilben-Derivaten oder 3-(4-Hydroxyphenyl)-1-(2,4,6-trihydroxyphenyl)-1-propanon (Phloretin) bei der Herstellung eines Medikaments zur Behandlung von Bewegungsstörungen durch die Modifikation der dopaminergen netto Aktivität des nigrostriatalen Reaktionsweges und durch Verabreichung in einer Abfolge oder durch gleichzeitige Verabreichung mit L-DOPA.

**15.** Die Verwendung von wenigstens einer hemmenden Verbindung für den Transport von L-DOPA aus den Nierenzellen, ausgewählt von Phenylbenzopyranderivaten, trans-Stilben-Derivaten oder 3-(4-Hydroxyphenyl)-1-(2,4,6-trihydroxyphenyl)-1-propanon (Phloretin) bei der Herstellung eines Medikamentes zur Steigerung der Zirkulationsniveaus des verabreichten L-DOPA in einem Subjekt.

**16.** Die Verwendung von wenigstens einer hemmenden Verbindung für den Transport von L-DOPA aus den Nierenzellen, ausgewählt aus Phenylbenzopyranderivaten, trans-Stilben-Derivaten oder 3-(4-Hydroxyphenyl)-1-(2,4,6-trihydroxyphenyl)-1-propanon (Phloretin) bei der Herstellung eines Medikamentes zur Verbesserung der Verfügbarkeit des verabreichten L-DOPA für das Gehirn eines Subjektes.

**17.** Die Verwendung gemäß einem der Ansprüche 13 bis 16, wobei die wenigstens eine hemmende Verbindung aus denjenigen, die in den Ansprüchen 2 bis 6 beschrieben sind, ausgewählt wird.

**18.** Die Verwendung gemäß einem der Ansprüche 13 bis 17, wobei die hemmende Verbindung in einer Menge vorliegt, die ca. 40 bis ca. 30,000µg/kg pro Dosis zur Verfügung stellt.

**19.** Die Verwendung gemäß einem der Ansprüche 13 bis 18, wobei die Behandlung ebenfalls die Verabreichung in einer Abfolge oder die gleichzeitige Verabreichung eines Inhibitors einer Aminosäuredecarboxylase oder eines Inhibitors der Catechol-O-Methyltransferase umfasst.

**20.** Die Verwendung gemäß Anspruch 19, wobei der Decarboxylaseinhibitor Benserazid oder Carbidopa umfasst und der Methyltransferaseinhibitor Entacapon oder Tolcapon umfasst.

**21.** Die Verwendung von wenigstens einer hemmenden Verbindung ausgewählt aus denjenigen, die in den Ansprüchen 1 bis 6 beschrieben wurden, bei der Herstellung eines Medikaments für die Behandlung der Parkinson Krankheit durch Hemmen der peripheren Decarboxylation des in einer Abfolge oder gleichzeitig verabreichten L-DOPA.

## Revendications

**1.** Composition pour le traitement de la maladie de Parkinson, la composition comprenant, en combinaison avec la lévodopa (L.DOPA), au moins un composé choisi parmi les dérivés du phényl-benzopyrane, les dérivés du trans-stilbène ou la 3-(4-hydroxyphényl)-1-(2,4,6-trihydroxyphényl)-1-propanone (phlorétine) pour bloquer le transfert vers l'extérieur des cellules rénales de la lévodopa, ce qui améliore la disponibilité de la lévodopa pour le cerveau.

**2.** Composition selon la revendication 1, dans laquelle le dérivé du phényl-benzopyrane comprend un composé flavonoïde.

**3.** Composition selon la revendication 2, dans laquelle le composé flavonoïde a la formule générale :

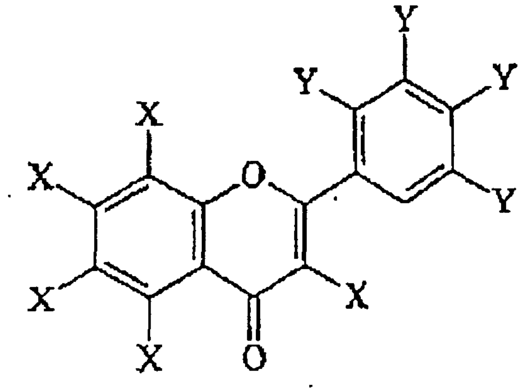

dans laquelle les groupes X sont les mêmes ou différents et sont choisis parmi H et OH, les groupes Y sont les mêmes ou différents et sont choisis parmi H et OR où R représente H, $CH_3$ et $CH_2$-Ph, incluant les dérivés 3-$C_6H_5$ ($Y_4$) correspondants.

**4.** Composition selon la revendication 1, dans laquelle le dérivé du trans-stilbène a la formule générale :

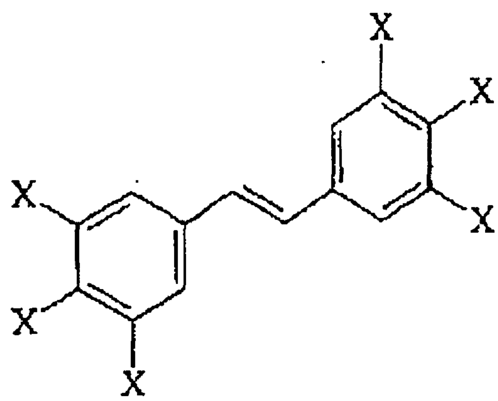

dans laquelle les groupes X sont les mêmes ou différents et sont H ou OH.

**5.** Composition selon la revendication 1, dans laquelle le composé bloquant est choisi parmi :

5,7-dihydroxy-2-(4-méthoxyphényl)-4H-1-benzopyran-4-one (acacétine),
5,7-dihydroxy-2-(4-hydroxyphényl)-4H-1-benzopyran-4-one (apigénine),
5,6,7-trihydroxy-2-phényl-4H-1-benzopyran-4-one (baicaléine),
5,7-dihydroxy-2-phényl-4H-1-benzopyran-4-one (chrysine),
([2R,3R])-2-[3,4-dihydroxyphényl]-3,4-dihydro-1[2H]benzopyran-3,5,7-triol ((-)-épicatéchin),2-(3,4-dihydroxyphényl)-3,7-dihydroxy-4H-1-benzopyran-4-one (fisétine),
5,7-dihydroxy-3-(4-hydroxyphényl)-4H-1-benzopyran-4-one (génistéine),
3,5,7-trihydroxy-2-(4-hydroxyphényl)-4H-1-benzopyran-4-one (kaempférol),
2-(2,4-dihydroxyphényl)-3,5,7-trihydroxy-4H-1-benzopyran-4-one (morine),
3,5,7-trihydroxy-2-(3,4,5-trihydroxyphényl)-4H-1-benzopyran-4-one (myricétine),
3-(4-hydroxyphényl)-1-(2,4,6-trihydroxyphényl)-1-propanone (phlorétine),
2-(3,4-dihydroxyphényl)-3,5,7-trihydroxy-4H-1-benzopyran-4-one (quercétine),
2-(3,4-dihydroxyphényl)-3,5,6,7-tétrahydroxy-4H-1-benzopyran-4-one (quercétagétine),
4'-benzyloxy-3',5'-diméthoxy-3,5,7-trihydroxyflavone,
3,7-dihydroxy-3',4',5'-triméthoxyflavone,3',4',7 ; 8-tétrahydroxyflavone,

3,5,7-trihydroxy-3',4',5'-triméthoxyflavone,3,3',4,5'-tétrahydroxy-trans-stilbène (piceatannol), trans-4-styrylphénol et 3,4',5-trihydroxy-trans-stilbène (resvératrol).

**6.** Composition selon l'une quelconque des revendications 1, 4 ou 5, dans laquelle le composé bloquant est le resvératrol.

**7.** Composition selon l'une quelconque des revendications précédentes comprenant en outre un inhibiteur de l'enzyme aminoacide décarboxylase et/ou un inhibiteur de l'enzyme catéchol-o-méthyl transférase.

**8.** Composition selon la revendication 7, dans laquelle ledit inhibiteur de l'aminoacide décarboxylase comprend du bensérazide ou de la carbidopa et ledit inhibiteur de la catéchol-o-méthyl transférase comprend de l'entacapone ou du tolcapone.

**9.** Composition selon l'une quelconque des revendications précédentes, la composition comprenant en outre des excipients inertes pharmaceutiquement acceptables.

**10.** Utilisation d'une composition selon l'une quelconque des revendications précédentes dans la préparation d'un médicament pour le traitement, ou la prévention de l'aggravation, d'une forme quelconque de la maladie de Parkinson.

**11.** Au moins un composé bloquant le transfert vers l'extérieur des cellules rénales de la lévodopa choisi parmi les dérivés du phényl-benzopyrane, les dérivés du trans-stilbène ou la 3-(4-hydroxyphényl)-1-(2,4,6-trihydroxyphényl)-1-propanone (phlorétine), en combinaison avec la lévodopa, destiné à une utilisation en thérapie par administration séquentielle ou simultanée.

**12.** Au moins un composé bloquant selon la revendication 11, dans lequel le au moins un composé bloquant est choisi parmi ceux décrits dans l'une quelconque des revendications 2 à 6.

**13.** Utilisation d'au moins un composé bloquant le transfert vers l'extérieur des cellules rénales de la lévodopa choisi parmi les dérivés du phényl-benzopyrane, les dérivés du trans-stilbène ou la 3-(4-hydroxyphényl)-1-(2,4,6-trihydroxyphényl)-1-propanone (phlorétine) dans la préparation d'un médicament pour le traitement, ou la prévention de l'aggravation, d'une forme quelconque de la maladie de Parkinson par administration séquentielle ou simultanée avec la lévodopa.

**14.** Utilisation d'au moins un composé bloquant le transfert vers l'extérieur des cellules rénales de la lévodopa choisi parmi les dérivés du phényl-benzopyrane, les dérivés du trans-stilbène ou la 3-(4-hydroxyphényl)-1-(2,4,6-trihydroxyphényl)-1-propanone (phlorétine) dans la préparation d'un médicament pour le traitement des troubles du mouvement par modification de l'activité dopaminergique nette de la voie strio-nigrale et par administration séquentielle ou simultanée avec la lévodopa.

**15.** Utilisation d'au moins un composé bloquant le transfert vers l'extérieur des cellules rénales de la lévodopa choisi parmi les dérivés du phényl-benzopyrane, les dérivés du trans-stilbène ou la 3-(4-hydroxyphényl)-1-(2,4,6-trihydroxyphényl)-1-propanone (phlorétine) dans la préparation d'un médicament pour augmenter les niveaux circulants de lévodopa administrée chez un sujet.

**16.** Utilisation d'au moins un composé bloquant le transfert vers l'extérieur des cellules rénales de la lévodopa choisi parmi les dérivés du phényl-benzopyrane, les dérivés du trans-stilbène ou la 3-(4-hydroxyphényl)-1-(2,4,6-trihydroxyphényl)-1-propanone (phlorétine) dans la préparation d'un médicament pour améliorer la disponibilité de la lévodopa administrée pour le cerveau d'un sujet.

**17.** Utilisation selon l'une quelconque des revendications 13 à 16, dans laquelle le au moins un composé bloquant est choisi parmi ceux décrits dans l'une quelconque des revendications 2 à 6.

**18.** Utilisation selon l'une quelconque des revendications 13 à 17, dans laquelle le composé bloquant est présent dans une quantité qui fournira environ 40 à environ 30 000 µg/kg par dose.

**19.** Utilisation selon l'une quelconque des revendications 13 à 18, dans laquelle le traitement comprend également l'administration simultanée ou séquentielle d'un inhibiteur de 1' aminoacide décarboxylase ou un inhibiteur de la

catéchol-o-méthyl transférase.

**20.** Utilisation selon la revendication 19, dans laquelle l'inhibiteur de la décarboxylase comprend du bensérazide ou de la carbidopa et l'inhibiteur de la méthyltransférase comprend de l'entacapone ou du tolcapone.

**21.** Utilisation d'au moins un composé bloquant choisi parmi ceux décrits dans l'une quelconque des revendications 1 à 6 dans la préparation d'un médicament pour le traitement de la maladie de Parkinson en bloquant la décarboxylation périphérique de la lévodopa administrée séquentiellement ou simultanément.

Fig 1

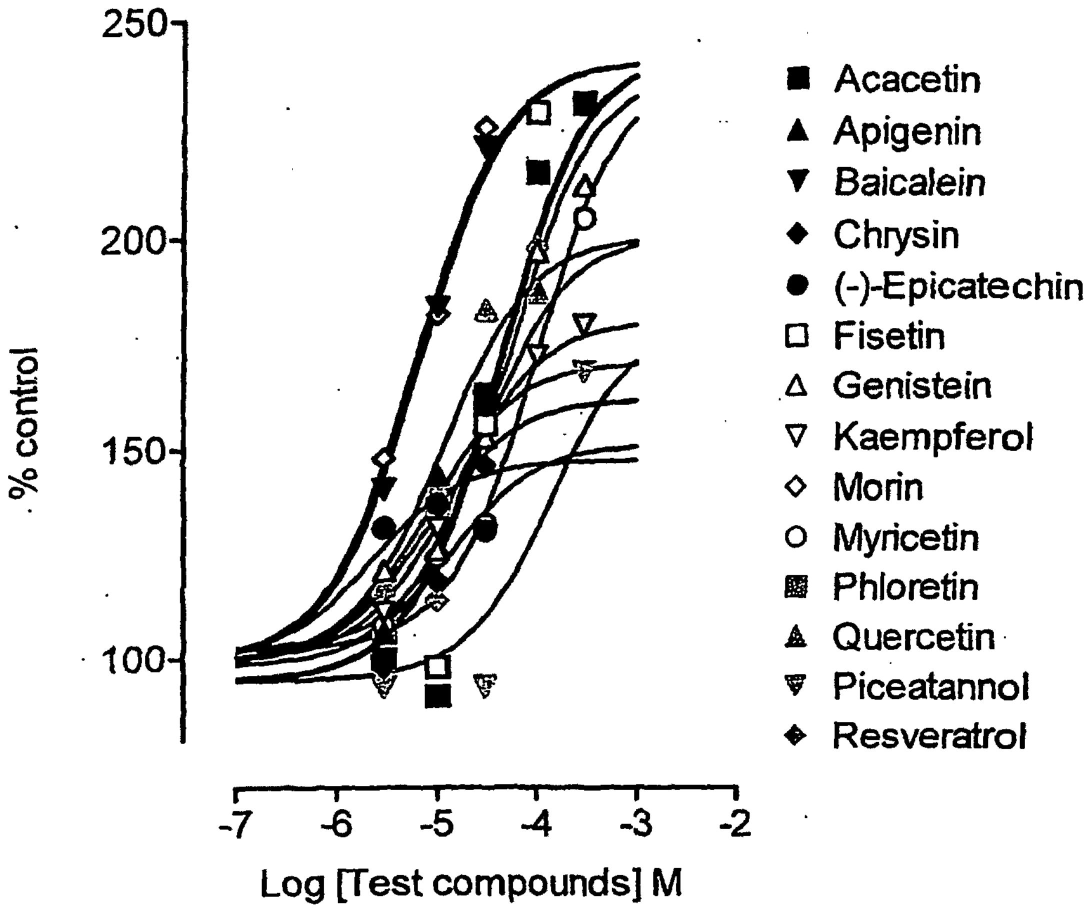
250
200
150
100
% control
-7
-6
-5
-4
-3
-2
Log [Test compounds] M
Acacetin
Apigenin
Baicalein
Chrysin
(-)-Epicatechin
Fisetin
Genistein
Kaempferol
Morin
Myricetin
Phloretin
Quercetin
Piceatannol
Resveratrol

Fig 2a

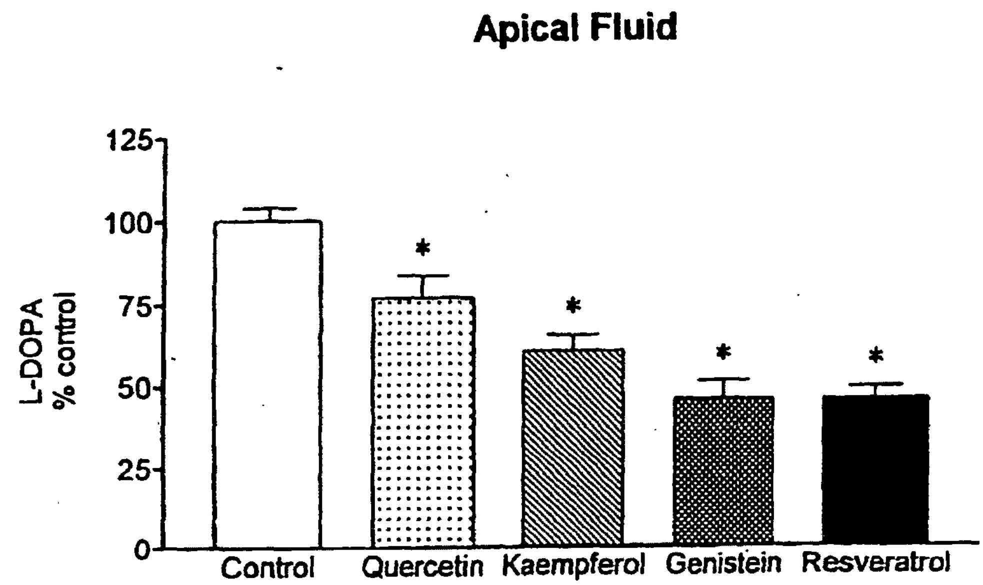


Fig 2b

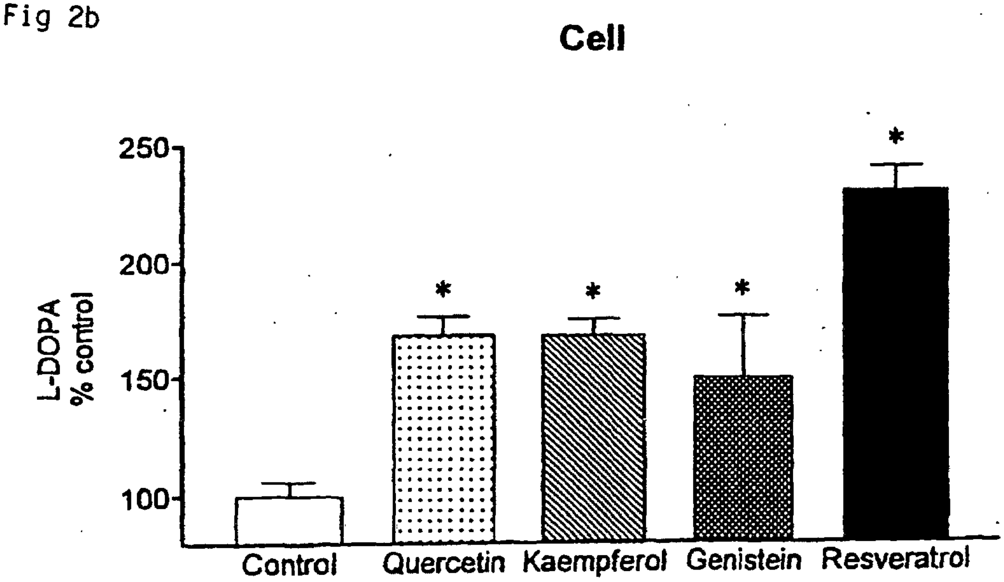


* P<0.05

Fig 3

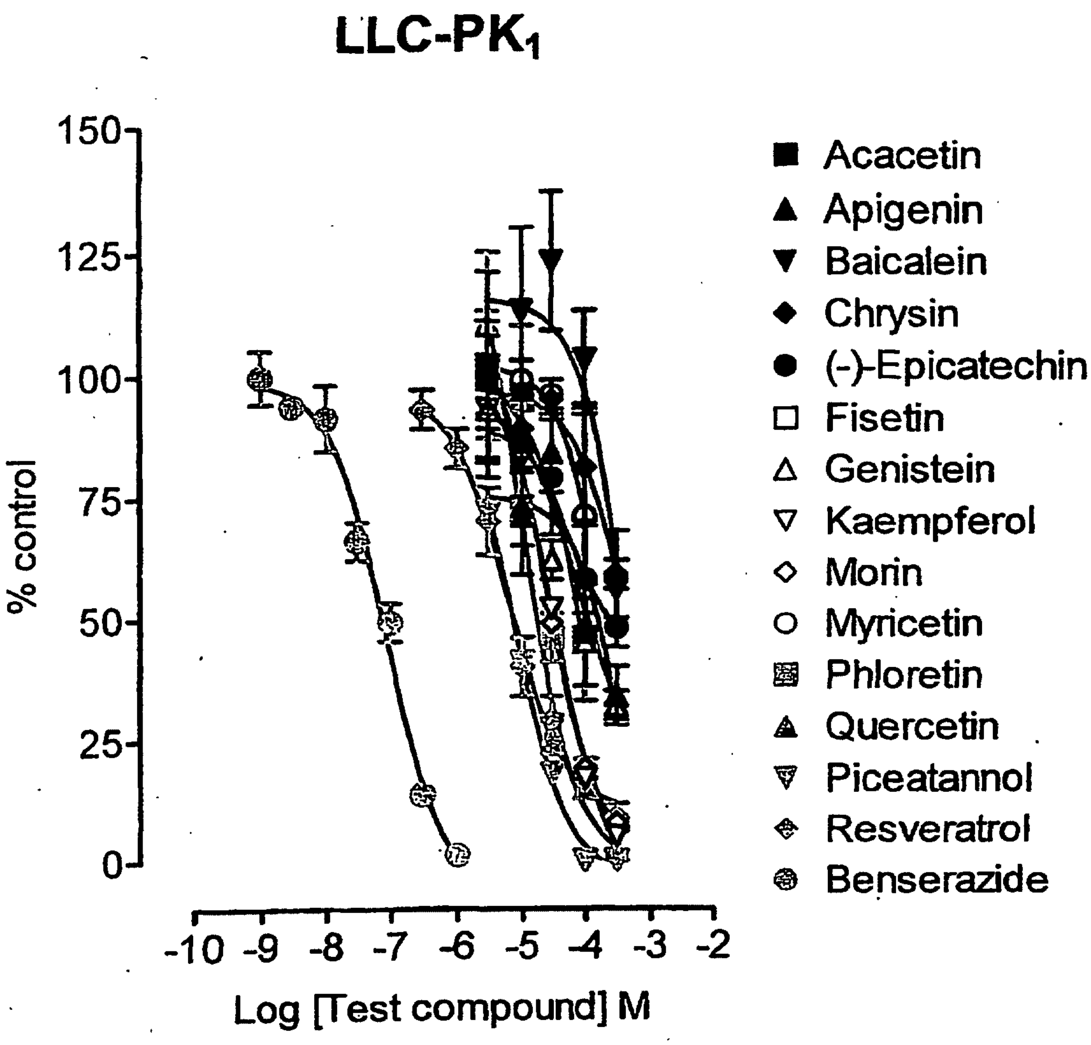
LLC-PK1
150
125
100
75
50
25
0
% control
-10 -9 -8 -7 -6 -5 -4 -3 -2
Log [Test compound] M
Acacetin
Apigenin
Baicalein
Chrysin
(-)-Epicatechin
Fisetin
Genistein
Kaempferol
Morin
Myricetin
Phloretin
Quercetin
Piceatannol
Resveratrol
Benserazide

Fig 4

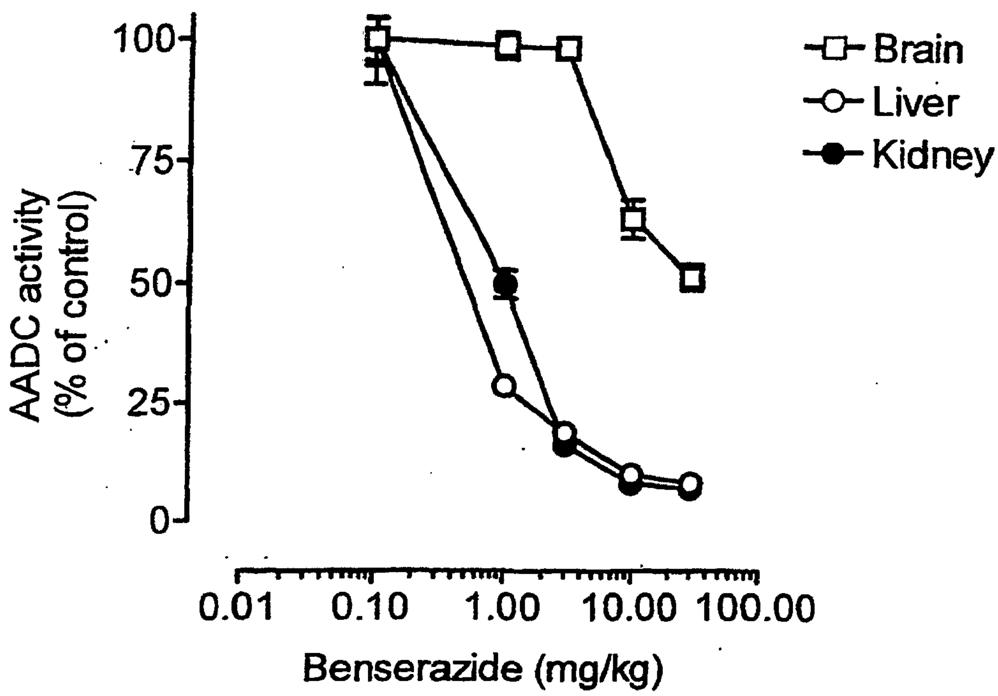
100
75
50
25
0
AADC activity
(% of control)
Brain
Liver
Kidney
0.01
0.10
1.00
10.00
100.00
Benserazide (mg/kg)

Fig 5

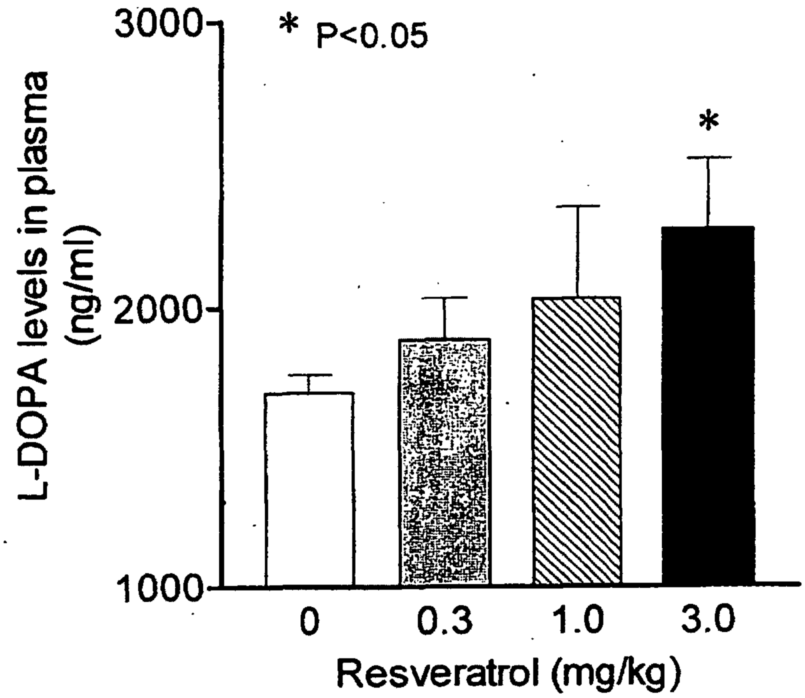
3000
2000
1000
* P<0.05
*
L-DOPA levels in plasma
(ng/ml)
0
0.3
1.0
3.0
Resveratrol (mg/kg)